# EUROPÄISCHE PATENTSCHRIFT

(11) **EP 3 599 460 B1**
(45) Veröffentlichungstag und Bekanntmachung des Hinweises auf die Patenterteilung: **14.04.2021**
(21) Anmeldenummer: 19188589.6
(22) Anmeldetag: 26.07.2019
(51) Int. Cl.: G01N 25/72, A62C 2/06

(54) **VERFAHREN ZUR ERFASSUNG MINDESTENS EINER EIGENSCHAFT EINES DÄMMSCHICHTBILDNERS**
METHOD FOR DETECTING AT LEAST ONE PROPERTY OF AN INTUMESCENT LAYER
PROCÉDÉ DE DÉTECTION D'AU MOINS UNE PROPRIÉTÉ D'UN REVÊTMENT INTUMESCENTE

(30) Priorität: 27.07.2018 DE 102018212622
(43) Veröffentlichungstag der Anmeldung: 29.01.2020
(73) Patentinhaber: Karlsruher Institut für Technologie, 76131 Karlsruhe (DE)
(72) Erfinder: Nagel, Sven, 76131 Karlsruhe (DE); Ummenhofer, Thomas, 76229 Karlsruhe (DE)
(74) Vertreter: Altmann Stößel Dick Patentanwälte PartG mbB

(56) Entgegenhaltungen:
- WO-A1-2018/020062
- WO-A2-2011/137547
- DE-A1-102014 108 399
- BILOTTA ANTONIO ET AL: "Tests on intumescent paints for fire protection of existing steel structures", CONSTRUCTION AND BUILDING MATERIALS, ELSEVIER, NETHERLANDS, Bd. 121, 10. Juni 2016 (2016-06-10), Seiten 410-422, XP029632401, ISSN: 0950-0618, DOI: 10.1016/J.CONBUILDMAT.2016.05.144

## Beschreibung

Die vorliegende Erfindung betrifft ein Verfahren zur Erfassung mindestens einer Eigenschaft eines Dämmschichtbildners. Die Erfindung ist insbesondere einsetzbar, um eine Leistungsfähigkeit von Dämmschichtbildern, mit welchen Bauteile, insbesondere Bauteile aus Stahl, von Bestandsgebäuden und Neubauten beschichtet sind, zu überprüfen. Es kann folglich eine Leistungsfähigkeit eines vorhandenen Brandschutzsystems und damit eine Sicherheit eines Bauwerks in einem Brandfall überprüft werden. Auch andere Anwendungen sind jedoch grundsätzlich denkbar.

### Stand der Technik

Reaktive Dämmschichtbildner werden in Deutschland seit den 1960er Jahren erfolgreich im baulichen Brandschutz eingesetzt und ermöglichen Architekten grundsätzlich Gestaltungsfreiräume beim Bauen mit sichtbarem Stahl. In einem Brandfall schäumen die reaktiven Dämmschichtbildner nach einer thermischen Aktivierung auf, isolieren die Bauteile und verzögern somit ein Überschreiten von kritischen Bauteiltemperaturen, welche zu einer Abnahme einer Festigkeit und Steifigkeit und einem Versagen der Bauteile führen können. Durch ein so gewonnenes Zeitfenster können eine Evakuierung und/oder eine zeitlich begrenzte Standsicherheit unter Brandeinwirkung möglich sein. Eine Leistungsfähigkeit des Dämmschichtbildners ist damit für eine Sicherheit von Bauwerken essentiell. Für weitere Einzelheiten wird auf IGSB-INFO 1, Brandschutzbeschichtungen im Stahlbau, herausgegeben von der Interessensgemeinschaft Stahl- und Brandschutzbeschichtung, Dezember 2014, verwiesen. Weiterhin wird auf den Beitrag "Mehr als zehn Jahre haltbar?" in der Zeitschrift "ausbau+fassade", Ausgabe 06.2017, Seiten 34-37, verwiesen.

Das Aufschäumverhalten des Dämmschichtbildners kann grundsätzlich durch klimatische und chemische Einflüsse erheblich bis hin zum Totalausfall beeinflusst werden. In Einzelfällen kann durch aufwändige Laborversuche, für welche im Rahmen von umfangreichen Sanierungsmaßnahmen Teile der Tragstruktur entnommen werden, auch nach 40 Jahren Standzeit noch eine ausreichende Leistungsfähigkeit des Dämmschichtbildners nachge wiesen werden. Bis 2006 erfolgte in Deutschland eine Zulassung der Dämmschichtbildner als Bauprodukt ausschließlich durch allgemeine bauaufsichtliche Zulassungen (abZ). Eine Dauerhaftigkeit des Dämmschichtbildners wurde durch ausgelagerte und dann im Brandversuch geprüften Vergleichsproben für einen Zeitraum von 10 Jahren sichergestellt. Da regelmäßige Untersuchungen sich auf visuelle Kontrollen beschränkten und keine Nutzungsdauer vorgeschrieben war, lag eine längere Anwendung im Graubereich.

Aktuell wird eine Zulassung der Dämmschichtbildner auf europäischer Ebene in Form von ETAs (European Technical Assessment) und der zugehörigen Vorschrift ETAG (European Technical Approval Guidelines) 018-2 geregelt. Erstmals wird eine vorgesehene Lebensdauer von 10 Jahren und einer alternativen Lebensdauer von 25 Jahren angegeben. Damit werden aktuell bei vielen Bauwerken die technischen und rechtlichen Grenzen bei Nutzung der reaktiven Dämmschichtbildner überschritten.

In der Leitlinie für die europäische technische Zulassung (ETAG) 018 Brandschutzprodukte, Teil 2: Reaktive Brandschutzbeschichtungen auf Stahlbauten, Ausgabe 2011, OIB-467-026/13, Anhang A, wird ein Zulassungsversuch beschrieben, bei welchem ein Probekörper mit den Abmessungen 5 mm x 300 mm x 200 mm mit dem zuzulassenden Dämmschichtbildner (einschließlich zugehöriger Deckbeschichtung und Grundierung) einseitig beschichtet wird. Die unbeschichtete Seite wird gedämmt und vor thermischer Einwirkung geschützt. Die beschichtete Seite wird einer vorgegeben Temperatur-Zeit-Kurve in einem stationären Brennofen ausgesetzt. Die Zeitdauer bis auf der unbeschichteten Seite eine Temperatur von 500 °C erreicht ist, stellt einen relevanten Parameter dar. Diese Verfahren werden zu unterschiedlichen, Alterungszuständen wiederholt.

In Jimenez, M., Bellayer, S., Naik, A., Bachelet, P., Dusquesne, S., Bqurbigot, S.: Topcoats versus Durability of an Intumescent Coating, I&EC research, 55, S. 9625-9632, 2016 wird ein vereinfachtes Verfahren zu den oben beschriebenen, in ETAG 018-2 gegebene Zulassungsversuchen vorgestellt. Auch in diesem Fall kommt ein stationärer Ofen und vergleichbare Probekörper zum Einsatz.

In DE 10 2012 214 379 A1 wird ein_Verfahren zur Herstellung eines Untergrundbelags, wobei eine erste Schicht bereitgestellt. Eine Sensorschicht wird auf die erste Schicht aufgebracht und eine zweite Schicht wird auf die Sensorschicht aufgebracht, sowie ein Untergrundbelag. Der Untergrundbelag weist eine erste Schicht, eine auf die erste Schicht aufgebrachte Sensorschicht und eine auf die Sensorschicht aufgebrachte zweite Schicht auf.

In DE 10 2012 103 975 B3 wird eine Vorrichtung zur Thermografie vorgeschlagen, Die Vorrichtung umfasst eine optische Detektionseinrichtung, die eine IR-Kamera umfasst. Die IR-Kamera ist eingerichtet, elektromagnetische Strahlung in einem zweiten Wellenlängenbereich zu detektieren. Weiterhin umfasst die Vorrichtung mindestens eine Anordnung von LEDs, die eingerichtet ist, zumindest in einem ersten, vom zweiten Wellenlängenbereich verschiedenen Wellenlängenbereich elektromagnetische Strahlung zu emittieren. Die Anordnung von LEDs gestattet eine in Bezug auf eine optische Achse der Detektionseinrichtung verstellbare Abstrahlungsrichtung der elektromagnetischen Strahlung der LEDs. Weiterhin umfasst die Vorrichtung einen dichroitischen Filter, beispielsweise einen dichroitischen Spiegel, der elektromagnetische Strahlung im ersten Wellenlängenbereich blockiert und elektromagnetische Strahlung im zweiten Wellenlängenbereich durchlässt. Der dichroitische Filter ist in der Detektionseinrichtung zwischen der IR-Kamera und einem zu prüfenden Prüfteil angeordnet.

Bilotta Antonio et al.: "Tests on intumescent paints for fire protection of existing steel structures", Construction and Building Materials 121, 2016, Seiten 410-422, zeigen Dickenmessungen und Adhäsionstests an mit Dämmschichtbildnern geschützten Stahlelementen, die aus einer bestehenden Struktur entnommen wurden. Die Tests wurden an Stahlbauteilen mit Original-Dämmschichtbildner und an Stahlbauteilen, die durch Auftragen eines neuen Dämmschichtbildners nach Entfernen der vorhandenen Farbe oder einfach durch Auftragen auf die vorhandene Farbe geschützt wurden, durchgeführt. Die Ergebnisse zeigen, dass der Original-Dämmschichtbildner nicht sehr effizient ist, wohingegen der restaurierte und der überstrichene Dämmschichtbildner eine ähnliche Leistung zeigten.

Trotz der durch diese Vorrichtungen und Verfahren bewirkten Verbesserungen ist nach wie vor ein Optimierungspotenzial vorhanden. Für bereits bekannte Verfahren werden grundsätzlich Bauteile aus einem Bestandsbauwerk entnommen und werden während einer Durchführung des Verfahrens zerstört. Es ist weiterhin grundsätzlich ein spezieller Probekörper mit definierten Dimensionen für eine Versuchsdurchführung notwendig.

### Aufgabe der Erfindung

Die Aufgabe der vorliegenden Erfindung besteht daher darin, die aufgeführten Nachteile und Einschränkungen des Standes der Technik zumindest teilweise zu überwinden. Insbesondere soll eine Entnahme von Bauteilen und somit eine Beeinflussung des Bauwerks vermieden werden. Weiterhin soll eine minimalinvasive Erfassung mindestens einer Eigenschaft eines Dämmschichtbildners ermöglicht werden.

### Offenbarung der Erfindung

Diese Aufgabe wird gelöst durch ein Verfahren zur Erfassung mindestens einer Eigenschaft eines Dämmschichtbildners. Für weitere Einzelheiten wird auf die Merkmale der abhängigen Ansprüche, die übrige Beschreibung und die Ausführungsbeispiele verwiesen.

Im Folgenden werden die Begriffe "haben", "aufweisen", "umfassen" oder "einschließen" oder beliebige grammatikalische Abweichungen davon in nicht-ausschließlicher Weise verwendet. Dementsprechend können sich diese Begriffe sowohl auf Situationen beziehen, in welchen, neben den durch diese Begriffe eingeführten Merkmalen, keine weiteren Merkmale vorhanden sind, oder auf Situationen, in welchen ein oder mehrere weitere Merkmale vorhanden sind. Beispielsweise kann sich der Ausdruck "A hat B", "A weist B auf", "A umfasst B" oder "A schließt B ein" sowohl auf die Situation beziehen, in welcher, abgesehen von B, kein weiteres Element in A vorhanden ist (d.h. auf eine Situation, in welcher A ausschließlich aus B besteht), als auch auf die Situation, in welcher, zusätzlich zu B, ein oder mehrere weitere Elemente in A vorhanden sind, beispielsweise Element C, Elemente C und D oder sogar weitere Elemente.

Weiterhin wird darauf hingewiesen, dass die Begriffe "mindestens ein" und "ein oder mehrere" sowie grammatikalische Abwandlungen dieser Begriffe, wenn diese in Zusammenhang mit einem oder mehreren Elementen oder Merkmalen verwendet werden und ausdrücken sollen, dass das Element oder Merkmal einfach oder mehrfach vorgesehen sein kann, in der Regel lediglich einmalig verwendet werden, beispielsweise bei der erstmaligen Einführung des Merkmals oder Elementes. Bei einer nachfolgenden erneuten Erwähnung des Merkmals oder Elementes wird der entsprechende Begriff "mindestens ein" oder "ein oder mehrere" in der Regel nicht mehr verwendet, ohne Einschränkung der Möglichkeit, dass das Merkmal oder Element einfach oder mehrfach vorgesehen sein kann.

Weiterhin werden im Folgenden die Begriffe "vorzugsweise", "insbesondere", "beispielsweise" oder ähnliche Begriffe in Verbindung mit optionalen Merkmalen verwendet, ohne dass alternative Ausführungsformen hierdurch beschränkt werden. So sind Merkmale, welche durch diese Begriffe eingeleitet werden, optionale Merkmale, und es ist nicht beabsichtigt, durch diese Merkmale den Schutzumfang der Ansprüche und insbesondere der unabhängigen Ansprüche einzuschränken. So kann die Erfindung, wie der Fachmann erkennen wird, auch unter Verwendung anderer Ausgestaltungen durchgeführt werden. In ähnlicher Weise werden Merkmale, welche durch "in einer Ausführungsform der Erfindung" oder durch "in einem Ausführungsbeispiel der Erfindung" eingeleitet werden, als optionale Merkmale verstanden, ohne dass hierdurch alternative Ausgestaltungen oder der Schutzumfang der unabhängigen Ansprüche eingeschränkt werden soll. Weiterhin sollen durch diese einleitenden Ausdrücke sämtliche Möglichkeiten, die hierdurch eingeleiteten Merkmale mit anderen Merkmalen zu kombinieren, seien es optionale oder nichtoptionale Merkmale, unangetastet bleiben.

Die vorliegende Erfindung betriff ein Verfahren zur Erfassung mindestens einer Eigenschaft eines Dämmschichtbildners, wobei gemäß Schritt a) des Verfahrens eine Vorrichtung zur Erfassung mindestens einer Eigenschaft eines Dämmschichtbildners, mit welchem ein Bauteil eines Bauwerks beschichtet ist, bereitgestellt wird. Die Vorrichtung umfasst mindestens ein Gehäuse. Das Gehäuse weist mindestens eine Prüföffnung auf, welche eingerichtet ist, einen mit dem Dämmschichtbildner beschichteten Prüfbereich auf dem Bauteil des Bauwerks von weiteren Bereichen des Bauteils abzugrenzen. Weiterhin umfasst die Vorrichtung mindestens eine Wärmequelle. Die Wärmequelle ist innerhalb des Gehäuses angeordnet und eingerichtet ist, dem Prüfbereich thermische Energie zuzuführen. Weiterhin umfasst die Vorrichtung mindestens einen Sensor. Der Sensor ist eingerichtet, eine durch die thermische Energie induzierte Änderung einer Eigenschaft des Dämmschichtbildners zu erfassen.

Der Begriff "Bauwerk" bezeichnet grundsätzlich eine beliebige von Menschen errichtete Konstruktion, die nur schwer lösbar mit dem Untergrund verbunden ist oder zumindest in einem ruhenden Kontakt zu dem Untergrund steht. Bei dem Bauwerk kann es sich insbesondere um ein Gebäude handeln. Der Begriff "Gebäude" bezeichnet grundsätzlich ein beliebiges Bauwerk, welches ein oder mehrere Räume einschließt, betreten werden kann und eingerichtet ist für einen Aufenthalt von Menschen, Tieren oder eine Lagerung von Gegenständen. Das Gebäude weist ein Dach auf. Weiterhin kann das Gebäude ein oder mehrere Wände und/oder einen Keller aufweisen. Insbesondere kann sich das Gebäude über der Erde erheben. Das Gebäude kann beispielswese ein Wohngebäude, ein Bürogebäude, eine Geschäftshaus, ein Kaufhaus, eine Fabrik, eine Werkstätte, eine Lagerhalle, eine Schule, eine Hochschule, eine Universität, ein Institut, ein Krankenhaus, ein Sanatorium, ein Altenheim, ein Pflegeheim, ein Kurgebäude, eine Strafvollzugsanstalt, ein Rathaus, ein Regierungsgebäude, eine Bibliothek, ein Museum, ein Konzerthaus, ein Opernhaus, ein Bürgerzentrum, eine Sporthalle, ein Schwimmbad, ein Kirchgebäude, ein Bahnhof, ein Flughafen oder ein Parkhaus sein. Auch andere Ausgestaltungen sind jedoch grundsätzlich denkbar.

Unter einem "Bauteil" ist im Rahmen der vorliegenden Erfindung grundsätzlich ein Einzelteil oder eine Komponente eines Bauwerks zu verstehen, aus welchem ein Bauwerk zusammengesetzt ist. Insbesondere kann es sich bei dem Bauteil um ein vorgefertigtes Teilstück für den Bau eines Bauwerks handeln. Bei dem Bauteil handelt es sich um eine geometrisch zusammenhängende Fläche oder Körper, die bzw. der grundsätzlich einen einheitlichen Aufbau und Konstruktion aufweist. Das Bauteil kann beispielsweise eine Wand, eine Stütze, eine Decke, ein Träger sein. Das Bauteil kann insbesondere aus Stahl hergestellt sein oder Stahl umfassen. Der Bauteil kann ein Stahlträger, d.h. ein tragendes Bauteil aus Stahl, sein. Der Stahlträger kann eingerichtet sein, um nach Fertigstellung des Bauwerks sichtbar zu sein.

Der Begriff "Dämmschichtbildner" bezeichnet grundsätzlich ein beliebiges Material oder einen beliebigen Beschichtungsstoff, welcher eingerichtet ist, bei einer Wärmeeinwirkung aufschäumen und eine im Bereich von Brandtemperaturen wärmeisolierende Dämmschicht zu bilden. Der Dämmschichtbildner kann vorzugsweise für eine Verbesserung einer Feuerwiderstandsfähigkeit von Bauteilen eingesetzt werden. In einem Brandfall kann der Dämmschichtbildner eingerichtet sein, nach einer thermischen Aktivierung aufzuschäumen, Bauteile zu isolieren und somit ein Überschreiten von kritischen Bauteiltemperaturen zu verzögern, welche zu einer Abnahme einer Festigkeit und einem Versagen der Bauteile führen. Durch ein so gewonnenes Zeitfenster kann eine Evakuierung bzw. eine zeitlich begrenzte Standsicherheit unter Brandeinwirkung möglich sein.

Insbesondere kann der Dämmschichtbildner als Beschichtung oder als Anstrich auf eine Stahlkonstruktion aufgebracht sein. Unter einer "Schicht" ist allgemein im Rahmen der vorliegenden Erfindung ein beliebiges Element mit einer flächigen Form und einer Dicke zu verstehen, welches auf einem anderen Objekt aufgebracht ist. Die Ausdehnung des Elements in lateraler Dimension kann die Dicke des Elements überschreiten, beispielsweise um einen Faktor von 10 bis 5000, vorzugsweise um einen Faktor von 200 bis 500.

Der Begriff "Beschichten" bezeichnet grundsätzlich einen beliebigen Vorgang, bei welchem eine Schicht auf eine Oberfläche eines anderen Objekts aufgebracht wird. Der Begriff "Beschichten" bezeichnet daher grundsätzlich einen beliebigen physikalischen und/oder chemischen Vorgang, mittels welchem eine Oberfläche eines beliebigen Körpers geändert oder modifiziert wird. Folglich unterscheiden sich physikalische und/oder chemische Eigenschaften des behandelten Körpers nach Durchführung der Beschichtung, insbesondere einer behandelten Oberfläche des Körpers, von physikalischen und/oder chemischen Eigenschaften des Körpers vor der Durchführung der Beschichtung. Durch das Beschichten der Oberfläche kann die beschichtete Oberfläche des beschichteten Körpers insbesondere eine chemische Zusammensetzung und/oder eine Oberflächenstruktur aufweisen, welche sich von einer chemischen Zusammensetzung und/oder einer Oberflächenstruktur des Körpers vor der Beschichtung unterscheiden. Das Beschichten kann beispielsweise mittels Walzen und/oder mittels einer Spritztechnik erfolgen. Auch andere Techniken sind grundsätzlich denkbar. Eine Beschichtung mit einem Dämmschichtbildner kann auch als Brandschutzanstrich oder Brandschutzbeschichtung bezeichnet werden. Der Begriff "Eigenschaft eines Dämmschichtbildners" bezeichnet grundsätzlich eine chemische und/oder physikalische Eigenschaft eines Dämmschichtbildners oder eine Änderung hiervon, welche sich durch mindestens eine Messgröße charakterisieren lässt. Die Eigenschaft des Dämmschichtbildners kann mittels des mindestens einen Sensors bestimmt werden. Der Begriff "Sensor", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung beziehen, welche mindestens eine Eigenschaft eines Stoffs, eines Systems oder eines Elements oder eine Änderung hiervon erfassen und in mindestens eine Messgröße umwandeln kann. Insbesondere kann der Sensor eingerichtet sein, um mindestens eine Eigenschaft mindestens des Dämmschichtbildners oder eine Änderung hiervon zu erfassen, beispielsweise mindestens eine chemische und/oder physikalische Eigenschaft oder deren Änderung. Unter einer Erfassung kann dabei allgemein eine qualitative oder insbesondere quantitative Erfassung der mindestens einen Eigenschaft verstanden werden, insbesondere eine Messung. Der Sensor kann eingerichtet sein, mindestens eine Messgröße oder eine Änderung einer Messgröße zu erfassen und mindestens ein Sensorsignal zu generieren, beispielsweise ein elektrisches Sensorsignal, aus welchem auf die Messgröße oder deren Änderung rückgeschlossen werden kann. Der Sensor kann insbesondere eine Schnittstelle aufweisen, welche ganz oder teilweise als Hardware und/oder Software ausgebildet sein kann. Der Sensor kann eingerichtet sein, um die Messgröße oder deren Änderung in ein elektrisches Signal, bevorzugt in eine elektrische Spannung und/oder einen elektrischen Strom umzuwandeln.

Der Sensor kann ausgewählt sein aus der Gruppe bestehend aus: einem Thermoelement; einer Wärmebildkamera; einem kontaktlosen Thermometer; einer Vorrichtung zur Schichtdickenbestimmung, insbesondere einer optischen, elektrischen oder mechanischen Vorrichtung zur Schichtdeckenbestimmung, insbesondere einem Laser- und/oder Infrarotentfernungsmesser, insbesondere einer Vorrichtung zur Schichtdickenbestimmung nach einem Keilschnittverfahren. Weiterhin kann der Sensor eingerichtet sein, einen optischen Vergleich mit einer integrierten Skala und/oder einer Bildkorrelation durchzuführen. Darüber hinaus kann die Schichtdicke des Dämmschichtbildners mittels eines Messschiebers bestimmt werden. Der Sensor kann in unterschiedlichen Höhen innerhalb des Prüfraums und/oder an dem Bauteil angebracht sein.

Der Sensor kann eingerichtet sein, während einer Bestrahlung des Prüfbereichs mit der Wärmequelle Temperatur-Zeit-Verläufe oder Schichtdicken-Zelt-Verläufe oder einzelne Messwerte zu definierten Zuständen an unterschiedlichen Stellen des Prüfbereichs zu bestimmen. Die Vorrichtung kann eingerichtet sein, bei bekannten Dämmschichtbildnern eine Bewertung einer Qualität des Dämmschichtbildners auf Grundlage von Datenbanken und Referenzproben, insbesondere nicht gealterten Referenzproben, erfolgen zu lassen. Weiterhin können die ermittelten Temperatur-Zeit-Verläufe als Grundlage einer rechnerischen Bewertung dienen. Hierbei auftretende Unsicherheiten können durch geeignete Sicherheitsfaktoren berücksichtigt werden.

Der Sensor kann innerhalb des Gehäuses angeordnet sein. Der Sensor und das Gehäuse können einstückig ausgebildet sein. Der Sensor kann eine integrale Komponente der Vorrichtung sein. Insbesondere kann der Sensor eingerichtet sein, um eine Messung, insbesondere eine Temperaturmessung und/oder eine Schichtdickenmessung durchzuführen, während dem Prüfbereich thermische Energie mittels der Wärmequelle zugeführt wird. Der Sensor kann derart innerhalb des Gehäuses angeordnet sein, dass der Sensor in einem direkten Kontakt mit mindestens einer Stelle des Prüfbereichs steht. Alternativ oder zusätzlich kann der Sensor innerhalb des Gehäuses in einem Abstand zu dem Prüfbereich angeordnet sein. Der Sensor kann daher eingerichtet sein, eine Messgröße kontaktfrei zu ermitteln.

Alternativ können der Sensor und das Gehäuse als separate Elemente ausgebildet sein. Insbesondere kann der Sensor eingerichtet sein, um eine Messung, insbesondere eine Schichtdickenmessung durchzuführen, bevor und/oder nachdem dem Prüfbereich thermische Energie mittels der Wärmequelle zugeführt wird bzw. wurde. Beispielsweise kann die Schichtdicke des Dämmschichtbildners mittels eines Messschiebers bestimmt werden.

Der Begriff "Gehäuse" bezieht sich im Sinne der vorliegenden Erfindung grundsätzlich auf ein beliebig geformtes Element, welches eingerichtet ist, um Bauteile der Vorrichtung ganz oder zumindest teilweise zu umschließen und um diese Bauteile weiterhin vor externen Einflüssen wie mechanischer Belastung und/oder Feuchtigkeit zu schützen. Das Gehäuse kann mehrteilig ausgestaltet sein. Mindestens ein erster Gehäuseteil kann mit mindestens einem zweiten Gehäuseteil verbunden sein. Insbesondere kann der erste Gehäuseteil mit dem zweiten Gehäuseteil verschraubt sein. Weiterhin können das erste Gehäuseteil und das zweite Gehäuseteil zusammengeklebt oder zusammengeschweißt sein.

Das Gehäuse kann zumindest teilweise einen Innenraum einschließen. Der Innenraum kann beispielsweise eine quaderförmige Grundform aufweisen. Auch andere Ausgestaltungen sind jedoch grundsätzlich denkbar. Der Innenraum kann als Einstell- und Erfassungskammer ausgebildet sein. Der Begriff "Einstell- und Erfassungskammer" bezeichnet im Sinne der vorliegenden Erfindung grundsätzlich einen beliebig ausgestalteten Hohlraum, welcher mindestens einen Einstellbereich und den mindestens einen Erfassungsbereich umfasst. Der Einstellbereich ist mit ein oder mehreren Gasen beaufschlagbar, wie nachfolgend noch näher ausgeführt wird. Insbesondere kann der Einstellbereich eingerichtet sein, um eine Gaszusammensetzung oder Temperatur innerhalb des Innenraums einzustellen. Der Erfassungsbereich kann insbesondere eingerichtet sein, um die mindestens eine Eigenschaft des Prüfbereichs zu erfassen. Die Begriffe "Einstellbereich" und "Erfassungsbereich" bezeichnen im Sinne der vorliegenden Erfindung grundsätzlich Teile der Einstell- und Erfassungskammer. Der Einstellbereich und der Erfassungsbereich können sich teilweise überlappen. Weiterhin können der Einstellbereich und der Erfassungsbereich integral ausgebildet sein. Auch andere Ausgestaltungen sind jedoch denkbar

Der Innenraum kann eine Messzelle sein und/oder eine Messzelle umfassen. Der Begriff "Messzelle", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung oder ein System beziehen, welches mindestens einen vollständig oder teilweise umschlossenen Innenraum aufweist, in welchem eine Messung der mindestens einen Eigenschaft erfolgt.

Das Gehäuse kann aus mindestens einem feuerfesten Material hergestellt sein. Insbesondere kann das Gehäuse aus mindestens einem Material hergestellt sein, ausgewählt aus der Gruppe bestehend aus: Brandschutzplatten; Calziumsilikatplatten; Mineralfaserplatten; Vermiculit; Scharmott; Metalle; Gipskarton; Holzkunststoff; Vakuumgehäuse.

Weiterhin kann das Gehäuse mindestens ein thermisches Isolationsmaterial aufweisen. Das thermische Isolationsmaterial kann eingerichtet sein, das Gehäuse von einer äußeren Umgebung des Gehäuses thermisch zu isolieren. Das thermische Isolationsmaterial kann mindestens ein Material umfassen ausgewählt, aus der Gruppe bestehend aus: Dämmwolle; Aerogelmatte; Blähglas; Calziumsilikatplatte; Faserdämmplatten; Vakuumdämmplatte Mineralwolle.

Weiterhin kann das Gehäuse ein oder mehrere Sichtfenster aufweisen. Das Sichtfenster kann insbesondere für eine Überwachung einer Prüfung des Dämmschichtbildners eingerichtet sein.

Die Vorrichtung kann insbesondere ein Handgerät sein. Der Begriff "Handgerät" bezeichnet grundsätzlich eine beliebige Vorrichtung, welche eingerichtet ist, von einem Menschen und zumindest weitgehend ohne Zuhilfenahme von weiteren Hilfsmitteln mit einer oder mit beiden Händen gehalten werden zu können. Die Vorrichtung kann daher auch als mobile Vorrichtung oder als tragbare Vorrichtung bezeichnet werden. Insbesondere kann das Handgerät eingerichtet sein, um mit einer Hand gehalten, transportiert und/oder positioniert zu werden. Insbesondere kann das Handgerät eingerichtet sein, um mit einer Hand des Benutzers in eine gewünschte Position gebracht zu werden, beispielsweise an eine Oberfläche des Bauteils des Gehäuses. Weiterhin kann das Handgerät eingerichtet sein, um mit einer anderen Hand des Benutzers in die gewünschte Position fixiert zu werden, beispielsweise mittels der Befestigungsvorrichtung, welche nachfolgend näher beschrieben wird.

Das Gehäuse kann eine quaderförmige, insbesondere eine würfelförmige Grundform aufweisen. Mindestens eine Abmessung des Gehäuses ausgewählt aus der Gruppe bestehend aus: einer Länge des Gehäuses, einer Breite des Gehäuses, einer Tiefe des Gehäuses kann 50 mm bis 500 mm, vorzugsweise von 100 mm bis 300 mm und besonders bevorzugt von 100 mm bis 200 mm betragen. Auch andere Dimensionen sind jedoch grundsätzlich denkbar. Das Gehäuse kann eine Dicke von 1 mm bis 500 mm, vorzugsweise von 5 mm bis 100 mm und besonders bevorzugt von 10 mm bis 60 mm aufweisen. Auch andere Dimensionen sind jedoch grundsätzlich denkbar. Das Gehäuse kann weiterhin eine Masse von 50 g bis 10000 g, vorzugsweise von 100 g bis 5000 g vorzugsweise von 1000 g bis 3000 g und besonders bevorzugt von 2000 g aufweisen.

Das Gehäuse kann optional weiterhin mindestens Befestigungselement aufweisen, welches eingerichtet ist, die Vorrichtung an das Bauteil des Bauwerks zu befestigen. Der Begriff "Befestigungselement" beschreibt ein beliebiges Element, welches eingerichtet ist, ein beliebiges Objekt an ein anderes Objekt anzubringen. Das Befestigungselement kann daher eingerichtet sein, das Objekt an das andere Objekt zu fixieren. Hierzu kann das Befestigungselement fest mit dem Objekt verbunden sein, insbesondere stoffschlüssig und/oder formschlüssig und/oder kraftschlüssig. Alternativ und/oder zusätzlich können das Objekt und das Befestigungselement einstückig ausgebildet sein. Der Begriff "Fixieren" bezeichnet im Sinne der vorliegenden Erfindung grundsätzlich, dass ein beliebiges Objekt in einer Position, insbesondere in einer gewünschten Position, derart festgehalten ist, dass ein Verschieben und/oder ein Verrutschen des Objekts von der gewünschten Position ganz oder zumindest teilweise reduziert oder sogar verhindert ist. Alternativ kann die Vorrichtung, insbesondere das Gehäuse, manuell durch einen Benutzer und/oder Anwender der Vorrichtung an das Gebäude gehalten werden.

Das Befestigungselement kann stoffschlüssig und/oder formschlüssig und/oder kraftschlüssig mit dem Gehäuse der Vorrichtung verbunden sein. Alternativ und/oder zusätzlich können das Befestigungselement und das Gehäuse einstückig ausgebildet sein. Das Gehäuse kann ein oder mehrere Seitenflächen aufweisen. Die Seitenfläche kann quer, insbesondere senkrecht zu einer Seite des Gehäuses angeordnet sein, welche die Prüföffnung aufweist. Das Befestigungselement kann an der Seitenfläche angeordnet sein. Das Befestigungselement kann eingerichtet sein, die Vorrichtung, insbesondere das Gehäuse, an das Bauteil, insbesondere einen Träger, insbesondere einen Stahlträger, zu befestigen, insbesondere zu fixieren. Das Befestigungselement kann daher eingerichtet, die Vorrichtung, insbesondere das Gehäuse, an das Bauwerk selbst zu befestigen. Das Befestigungselement kann insbesondere eines oder mehrere der folgenden Elemente zur Befestigung des Gehäuses an das Bauteil aufweisen: eine Klemme, einen Magnetfuß, ein Stativ, einen Spanngurt, eine Schraube, einen Saugnapf, ein Seil, einen Schnellspanner.

Der Begriff "Öffnung" im Sinne der vorliegenden Erfindung bezeichnet einen Durchbruch, eine Durchgangsöffnung oder eine Aussparung an einer Außenwand des Gehäuses. Wie bereits oben dargestellt, kann das Gehäuse einen Innenraum aufweisen und die Öffnung kann eingerichtet sein, den Innenraum des Gehäuses mit einer äußeren Umgebung des Gehäuses zu verbinden. Der Begriff "Prüföffnung" im Sinne der vorliegenden Erfindung bezeichnet demnach eine Öffnung an der Außenwand des Gehäuses, welche eine Oberfläche eines Prüfkörpers, insbesondere eine Oberfläche des Bauteils des Bauwerks mit dem Innenraum des Gehäuses verbindet. Insbesondere kann die Prüföffnung eingerichtet sein, den Prüfbereich des Bauteils des Bauwerks, welcher nachfolgend noch näher beschrieben wird, mit dem Innenraum des Gehäuses zu verbinden. Die Prüföffnung kann eingerichtet sein, dem Prüfbereich thermische Energie von der Wärmequelle durch die Prüföffnung zuzuführen. Der Sensor kann, wie oben ausgeführt, innerhalb des Gehäuses angeordnet sein und der Sensor kann eingerichtet sein, mindestens eine Messgröße des Prüfbereichs zu erfassen. Der Sensor kann der Prüföffnung gegenüberliegend angeordnet sein. Die Prüföffnung kann eine runde Grundform aufweisen. Insbesondere kann die Prüföffnung eine kreisrunde Grundform. Auch andere Grundformen, wie beispielsweise ovale oder eckige Grundformen, sind jedoch grundsätzlich denkbar. Die Prüföffnung kann einen Durchmesser von 10 mm bis 100 mm, vorzugsweise von 20 mm bis 80 mm und besonders bevorzugt von 30 mm bis 60 mm aufweisen.

Der Begriff "Bereich" bezeichnet grundsätzlich einen Teil oder eine Sektion eines beliebigen Objekts. Insbesondere kann es sich bei dem Bereich um einen Teil oder eine Sektion einer Oberfläche des Objekts handeln. Der Bereich kann wiederrum in mehrere Teilbereiche untergliedert sein. Die Teilbereiche können zusammenhängende Bereiche sein, welche in direktem Kontakt zueinander stehen. Alternativ können die Teilbereiche voneinander getrennt ausgebildet sein, wobei die Teilbereiche voneinander beabstandet sind. Der Begriff "Prüfbereich" bezeichnet im Rahmen der vorliegenden Erfindung einen Bereich des Bauteils des Gehäuses, welcher eingerichtet ist, thermische Energie der Wärmequelle, welche nachfolgend noch näher beschrieben wird, zugeführt zu bekommen. Weiterhin bezeichnet der Begriff "Prüfbereich" im Rahmen der vorliegenden Erfindung einen Bereich des Bauteils des Gehäuses, von welchem die Erfassung der mindestens einen Eigenschaft des Dämmschichtbildners erfolgt. Der Prüfbereich kann einen Bereich des Bauteils umfassen, welche ganz oder zumindest teilweise mit dem Dämmschichtbildner beschichtet ist.

Der Prüfbereich kann durch die Prüföffnung begrenzt sein. Insbesondere kann der Prüfbereich eine Grundform aufweisen, welche der Grundform der Prüföffnung entspricht. Der Prüfbereich kann einen Durchmesser von 10 mm bis 100 mm, vorzugsweise von 20 mm bis 80 mm und besonders bevorzugt von 30 mm bis 60 mm aufweisen. Auch andere Dimensionen sind jedoch grundsätzlich denkbar.

Die Prüföffnung ist, wie oben bereits ausgeführt, eingerichtet, dem Prüfbereich auf dem Bauteil des Bauwerks von weiteren Bereichen des Bauteils abzugrenzen. Der Begriff "abgrenzen" im Sinne der vorliegenden Erfindung bezieht sich insbesondere auf eine thermische und/oder mechanische Entkopplung des Prüfbereichs von anderen Bereichen des Bauteils. Insbesondere kann die Prüföffnung eingerichtet sein, den innerhalb des Prüfbereichs angeordneten Dämmschichtbildner von einem außerhalb des Prüfbereichs angeordneten Dämmschichtbildner zu trennen. Die Prüföffnung des Gehäuses kann mindestens eine zumindest teilweise umlaufende Schneidvorrichtung umfassen, welche eingerichtet ist, eine erste Dämmschichtbildnerbeschichtung innerhalb des Prüfbereichs von einer zweiten Dämmschichtbildnerbeschichtung außerhalb des Prüfbereichs thermisch und/oder mechanisch zu entkoppeln. Die Schneidvorrichtung kann eingerichtet sein, eine Aussparung zwischen der ersten Dämmschichtbildnerbeschichtung und der zweiten Dämmschichtbilderbeschichtung zu bilden. Die Schneidvorrichtung kann insbesondere mindestens eine Klinge und/oder mindestens ein Schneidrad und/oder mindestens einen Pin, insbesondere eine Schneidnadel, sein oder umfassen.

Die Bezeichnungen "erste Dämmschichtbildnerbeschichtung" und "zweite Dämmschichtbildnerbeschichtung" sind als reine Bezeichnungen anzusehen, ohne eine Reihenfolge oder Rangfolge anzugeben und beispielsweise ohne die Möglichkeit auszuschließen, dass mehrere Arten von ersten Dämmschichtbildnerbeschichtungen und mehrere Arten von zweiten Dämmschichtbildnerbeschichtungen oder jeweils genau eine Art vorgesehen sein kann. Weiterhin können zusätzliche Dämmschichtbildnerbeschichtungen, beispielsweise eine oder mehrere dritte Dämmschichtbildnerbeschichtungen vorhanden sein. Die erste Dämmschichtbildnerbeschichtung und die zweite Dämmschichtbildnerbeschichtung können jeweils einen gleichen Dämmschichtbildner aufweisen.

Zwischen der ersten Dämmschichtbildnerbeschichtung und der zweiten Dämmschichtbilderbeschichtung kann sich ein Dämmschichtbildner-freier Bereich ausbilden. Die Klinge kann sich quer, insbesondere senkrecht, zu dem Prüfbereich erstrecken. Die Klinge kann an einem Ende der Prüföffnung angeordnet sein, welches dem Bauteil zugewandt ist. Die Klinge kann ein oder mehrere Schneiden aufweisen, welche eingerichtet sind, den Dämmschichtbildner von der Oberfläche des Bauteils abzutragen sodass sich die oben beschriebene Aussparung ausbildet.

Der Begriff "Wärmequelle" bezeichnet im Rahmen der vorliegenden Erfindung ein beliebiges technisches Gerät, welches eingerichtet ist, Wärme an eine Umgebung des technischen Geräts abzugeben. Der Begriff "thermische Energie" bezeichnet grundsätzlich eine Energie, welche einer ungeordneten Bewegung von Atomen oder Molekülen eines Stoffes gespeichert ist. Die thermische Energie kann daher auch als Wärmeenergie bezeichnet werden. Die Wärmequelle kann eingerichtet sein, den Prüfbereich des Bauteils aufzuheizen. Der Begriff "Aufheizen" bezeichnet allgemein einen beliebigen Vorgang, bei welchem ein Medium, beispielsweise ein gasförmiges Medium, ein flüssiges Medium und/oder ein Medium aus einer Mischung aus einem gasförmigen Medium mit einem flüssigen Medium, erhitzt oder erwärmt wird. Alternativ oder zusätzlich kann das Aufheizen des Mediums durch Beaufschlagung mit Wärmestrahlung erfolgen. Weiterhin kann die Vorrichtung eine Leistungssteuerungseinheit umfassen. Die Leistungssteuerungseinheit kann eingerichtet sein, eine Leistung der Wärmequelle zu regeln.

Die Wärmequelle ist, wie oben beschrieben, innerhalb des Gehäuses angeordnet. Die Wärmequelle kann insbesondere an einer Innenseite einer Wand des Gehäuses angeordnet sein. Ein Abstand zwischen der Wärmequelle und der Prüföffnung kann variierbar sein. Die Wärmequelle kann ausgewählt sein aus der Gruppe bestehend aus: Infrarotstrahler; Gasbrenner.

Die Vorrichtung kann weiterhin mindestens eine Absaugvorrichtung umfassen, welche eingerichtet ist, Gase aus dem Gehäuse in eine äußere Umgebung des Gehäuses zu transportieren. Darüber hinaus kann die Vorrichtung weiterhin mindestens eine Gaszuführungsvorrichtung umfassen, welche eingerichtet ist, Gase in einen Innenraum des Gehäuses zu transportieren, insbesondere Sauerstoff. Durch eine Anreicherung mit Sauerstoff oder eines anderen Gases kann eine Entzündung von sich innerhalb des Innenraums des Gehäuses auftretenden Gasen vermieden werden. Der Begriff "Gas" bezeichnet ein grundsätzlich beliebiges gasförmiges Element, eine grundsätzlich beliebige gasförmige Verbindung oder auch um ein grundsätzlich beliebiges Gemisch gasförmiger Elemente und/oder Verbindungen beziehen.

Wie bereits erwähnt, betrifft die vorliegende Erfindung ein Verfahren zur Erfassung mindestens einer Eigenschaft eines Dämmschichtbildners, mit welchem ein Bauteil eines Bauwerks beschichtet ist. Das Verfahren umfasst hierbei die Verfahrensschritte, welche im Folgenden im Einzelnen beschrieben werden. Die Verfahrensschritte können insbesondere in der vorgegebenen Reihenfolge durchgeführt werden. Eine andere Reihenfolge ist jedoch ebenfalls denkbar. Weiterhin können ein oder mehrere Verfahrensschritte gleichzeitig oder zeitlich überlappend durchgeführt werden. Weiterhin können einer, mehrere oder alle der Verfahrensschritte einfach oder wiederholt durchgeführt werden. Das Verfahren kann darüber hinaus noch weitere Verfahrensschritte umfassen.

Das Verfahren umfasst die folgenden Schritte:
a) Bereitstellen der Vorrichtung wie sie bereits beschrieben wurde oder im Folgenden noch beschrieben wird;
b) Befestigen der Vorrichtung an das Bauteil des Bauwerks, insbesondere mittels des Befestigungselements, derart, dass die Prüföffnung des Gehäuses einen mit dem Dämmschichtbildner beschichteten Prüfbereich auf dem Bauteil des Bauwerks von weiteren Bereichen des Bauteils abgrenzt;
c) Zuführen von thermischer Energie an dem Prüfbereich mittels der Wärmequelle; und
d) Erfassen mindestens einer durch die thermische Energie induzierten Änderung einer Eigenschaft des Dämmschichtbildners mittels des Sensors.

Nach Durchführen des Schritts c) oder d) kann der Prüfbereich mit einem weiteren Dämmschichtbildner beschichtet werden. Der weitere Dämmschichtbildner kann identisch sein zu dem Dämmschichtbildner.

### Vorteile der Erfindung

Die vorgeschlagenen Vorrichtungen und die vorgeschlagenen Verfahren weisen über bekannten Vorrichtungen und Verfahren zahlreiche Vorteile auf

Bei aus dem Stand der Technik bekannten Vorrichtungen und Verfahren kommt üblicherweise ein stationärer Ofen zum Einsatz. Hierfür wird üblicherweise ein Probekörper eines Bauteils zerstörend aus einem Bestandstragwerk entnommen und während des Verfahrens zerstört.

Durch das Verfahren gemäß der vorliegenden Erfindung sind grundsätzlich keine speziellen Probekörper für die Durchführung des Verfahrens notwendig. Die Vorrichtung kann direkt am Bauteil bzw. am Bauwerk angebracht werden. Das Verfahren kann direkt am Bauteil bzw. am Bauwerk durchgeführt werden. Eine Entnahme von Probekörpers aus dem Bauteil kann entfallen. Dadurch wird das Bauwerk nicht zerstört. Das Verfahren stellt folglich ein minimalinvasives in-situ Prüfverfahren dar. Mithilfe der erfindungsgemäßen Vorrichtung und des erfindungsgemäßen Verfahrens kann eine Entwicklung von Dämmschichtbildnern untersucht werden. Weiterhin kann mithilfe der erfindungsgemäßen Vorrichtung und des erfindungsgemäßen Verfahrens eine ausreichende Leistungsfähigkeit des Dämmschichtbildners untersucht werden. Eine Prüfung des Dämmschichtbildners kann vor Ort stattfinden. Weiterhin ist eine Prüfung grundsätzlich zumindest nahezu unabhängig von einer Geometrie des Bauteils.

### Kurze Beschreibung der Figuren

Bevorzugte Ausführungsbeispiele der vorliegenden Erfindung sind in den Figuren dargestellt und werden in der nachfolgenden Beschreibung ohne Beschränkung der Allgemeinheit näher erläutert. Hierbei zeigen:
- Figuren 1A und 1B: ein Ausführungsbeispiel einer Vorrichtung zur Bereitstellung ein einem erfindungsgemäßen Verfahren in einer schematischen Darstellung (Figur 1A) und eine schematische Darstellung eines Bauwerks (Figur 1B); und
- Figur 2: Temperatur-Zeit-Verläufe zur Erfassung mindestens einer Eigenschaft eines Dämmschichtbildners.

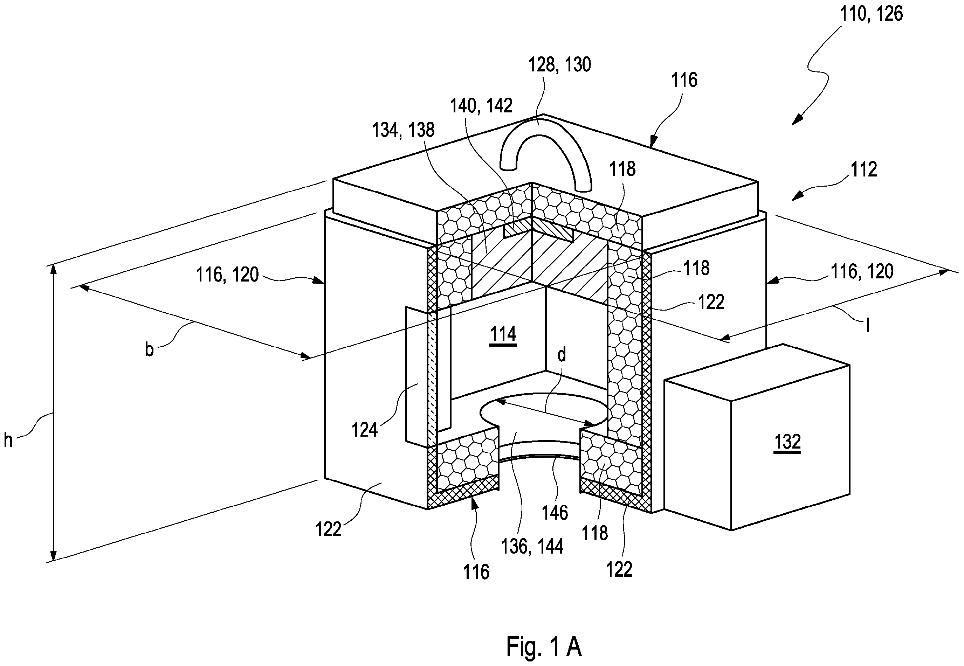

In Figur 1A ist eine Vorrichtung 110 zur Erfassung mindestens einer Eigenschaft eines Dämmschichtbildners, die gemäß Schritt a) des erfindungsgemäßen Verfahrens bereitgestellt wird, in einer schematischen Darstellung gezeigt.

Die Vorrichtung 110 umfasst mindestens ein Gehäuse 112. Das Gehäuse 112 kann eine quadratische, rechteckig oder runde Grundform aufweisen. Weiterhin kann das Gehäuse 112 einen Innenraum 114 einschließen. Das Gehäuse 112 kann mehrere Wände 116 aufweisen. Die Wände 116 können aus einem thermischen Isolationsmaterial 118 hergestellt sein. kann aus mindestens einem hergestellt sein. Seitenwände 120 des Gehäuses 112 können zusätzlich ein feuerfestes Material 122 aufweisen. Das feuerfeste Material 122 kann eine äußere Hülle des Gehäuses 112 bilden. Weiterhin kann das Gehäuse 112 ein oder mehrere Sichtfenster 124 aufweisen. Das Sichtfenster 124 kann für eine Überwachung einer Prüfung des Dämmschichtbildners eingerichtet sein.

Die Vorrichtung kann insbesondere ein Handgerät 126 sein. Insbesondere kann das Handgerät 126 eingerichtet sein, um mit einer Hand gehalten, transportiert und/oder positioniert zu werden. Hierfür kann das Handgerät 126 eine Tragevorrichtung 128, insbesondere einen Griff 130 aufweisen. Das Gehäuse kann eine Länge I, eine Breite b und eine Tiefe t aufweisen. Die Länge, die Breite und die Tiefe können jeweils 150 mm betragen.

Das Gehäuse 112 kann darüber hinaus mindestens eine Befestigungselement 132 aufweisen, welches eingerichtet ist, die Vorrichtung 112, insbesondere das Gehäuse 112 an das Bauteil zu befestigen. Das Befestigungselement 132 kann stoffschlüssig und/oder formschlüssig und/oder kraftschlüssig mit dem Gehäuse 112 der Vorrichtung verbunden sein. Insbesondere kann das Befestigungselement 132 an einer der Seitenwände 120 des Gehäuses 112 angeordnet sein.

Weiterhin weist die Vorrichtung 110 mindestens eine Wärmequelle 134 auf. Die Wärmequelle 134 ist innerhalb des Gehäuses 112 angeordnet. Insbesondere kann die Wärmequelle 134 innerhalb des Innenraums 114 des Gehäuses 112 angeordnet sein. Die Wärmequelle 134 kann an einer der Wände 116 des Gehäuses 112 befestigt sein. Die Wärmequelle 134 kann eine Prüföffnung 136 des Gehäuses 112 gegenüberliegend angeordnet sein. Die Wärmequelle 134 kann insbesondere eine Infrarot-Wärmequelle 138 sein. Die Vorrichtung 110 umfasst weiterhin mindestens einen Sensor 140 zur Erfassung mindestens einer Eigenschaft des Dämmschichtbildners. Der Sensor 140 kann beispielsweise eine Wärmebildkamera 142 sein. Der Sensor 140 kann ebenfalls innerhalb des Gehäuses 112, insbesondere in dem Innenraum 114 angeordnet sein. Der Sensor 140 kann eine Prüföffnung 136 des Gehäuses 112 gegenüberliegend angeordnet sein. Der Sensor 140 kann eingerichtet sein, während einer Bestrahlung des Prüfbereichs mit der Wärmequelle Temperatur-Zeit-Verläufe oder Schichtdicken-Zelt-Verläufe an unterschiedlichen Stellen des Prüfbereichs zu bestimmen.

Weiterhin weist die Vorrichtung die Prüföffnung 136 auf. Die Prüföffnung 136 kann als Durchgangsöffnung 144 einer der Wände 116 des Gehäuses 112 ausgebildet sein. Die Prüföffnung 136 kann eingerichtet sein, den Innenraum 114 des Gehäuses 112 mit einer äußeren Umgebung des Gehäuses 112 zu verbinden. Insbesondere kann die Prüföffnung 136 eingerichtet sein, den Prüfbereich des Bauteils mit dem Innenraum 114 des Gehäuses 112 zu verbinden. Die Prüföffnung 136 kann eingerichtet sein, dem Prüfbereich thermische Energie von der Wärmequelle 134 durch die Prüföffnung 134 zuzuführen. Der Sensor 140 kann eingerichtet sein, mindestens eine Messgröße des Prüfbereichs zu erfassen.

Die Prüföffnung 136 kann eine runde Grundform aufweisen. Die Prüföffnung 136 kann insbesondere einen Durchmesser von 40 mm aufweisen. Die Prüföffnung 136 des Gehäuses 112 kann mindestens eine zumindest teilweise umlaufende Schneidvorrichtung146 umfassen, welche eingerichtet ist, eine erste Dämmschichtbildnerbeschichtung innerhalb des Prüfbereichs von einer zweiten Dämmschichtbildnerbeschichtung außerhalb des Prüfbereichs thermisch und/oder mechanisch zu entkoppeln.

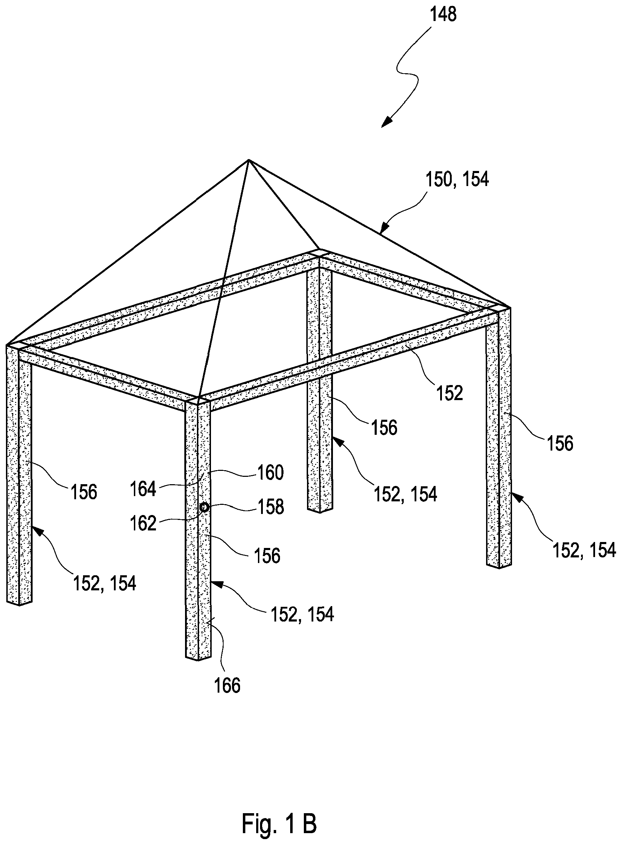

Figur 1B zeigt eine schematische Darstellung eines exemplarischen Bauwerks 148. Das Bauwerk kann ein Dach 150 aufweisen und mehrere Stahlträger 152. Das Dach 150 und die Stahlträger 152 können als Bauteile 154 bezeichnet werden. Die Stahlträger oder Stützen können jeweils mit einem Dämmschichtbildner 156 beschichtet sein. Die Vorrichtung 110, wie in Figur 1A darstellt, kann eingerichtet sein, um auf einen der Strahlträger 152 befestigt zu werden. Die Prüföffnung 136 der Vorrichtung 110 kann eingerichtet sein, eine einen mit dem Dämmschichtbildner 156 beschichteten Prüfbereich 158 auf dem Stahlträger 152 von weiteren Bereichen 160 des Stahlträgers 152 abzugrenzen. Der Stahlträger 152 kann eine erste Dämmschichtbildnerbeschichtung 162 innerhalb des Prüfbereichs 158 und eine zweite Dämmschichtbildnerbeschichtung 164 außerhalb des Prüfbereichs 158 aufweisen. Die erste Dämmschichtbildnerbeschichtung 162 und die zweite Dämmschichtbildnerbeschichtung 164 können jeweils einen gleichen Dämmschichtbildner 152 aufweisen und zwei voneinander räumlich getrennte oder verschiedene Bereiche auf einer Oberfläche 166 des Stahlträgers 152 darstellen.

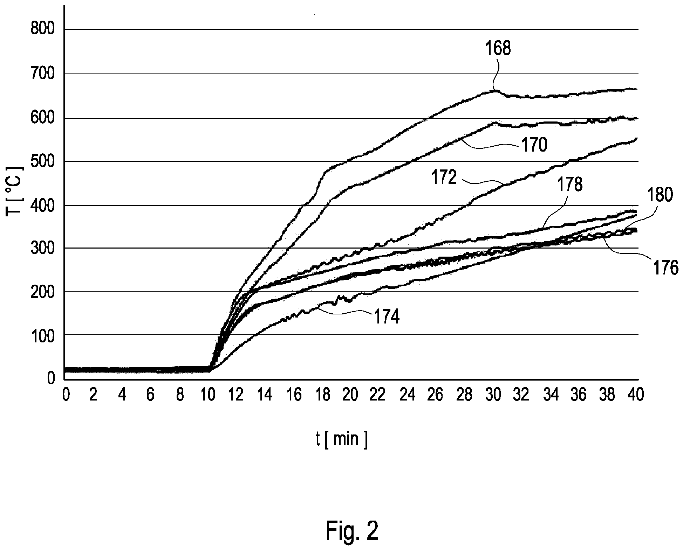

Figur 2 zeigt Temperatur-Zeit-Verläufe zur Erfassung mindestens einer Eigenschaft eines Dämmschichtbildners. Die Proben wurden aus Normproben nach ETAG 02 entnommen. Dazu wurde ein kreisförmiger Anriss in eine Dämmschichtbildnerbeschichtung auf einem Bauteil eingebracht. Eine Ausgangsschichtdicke der Dämmschichtbildnerbeschichtung wurde durch eine Vorrichtung zur Dickenmessung nach dem Keilschnittverfahren, insbesondere mittels eines Paint Borers, gemessen. Auf den abgetrennten Bereich wurde die Vorrichtung gestellt. Die Probe wurde mit einer Brandschutzplatte abgedeckt. Ein eingesetzter Infrarotstrahler für 10 Minuten vorgewärmt. Anschließend wurde die Abdeckung entfernt. Ein Temperatur wurde auf einer Probenrückseite durch ein Thermoelement gemessen. Der Versuch wurde an unterschiedlich künstlich gealterten Proben wiederholt. Nach dem Versuch wurde die Schichtdicke mit einem Messschieber gemessen.

Die Linien 168, 170 zeigen jeweils Temperatur-Zeit-Verläufe von einer Probe, welche in einer Salzsprühnebelanlage präpariert würde. Die Behandlung erfolgte über einen Zeitraum von 10 Tagen bei einer Temperatur von 35 °C mit einer Natriumkonzentration von 50 g/l. Linie 172 zeigt einen Temperatur-Zeit-Verlauf einer Probe, welche 112 mal mit einem Zyklus behandelt wurde, welcher ein fünfstündiges Trocken mit UV Beleuchtung und eine stündige Behandlung mit Sprühwasser umfasst. Bei den Linien 174, 176 handelt es sich um Temperatur-Zeit-Verläufe von intakten Proben mit einer Deckbeschichtung. Bei den Linien 178, 180 handelt es sich um Temperatur-Zeit-Verläufe von Proben ohne künstliche Alterung.

Es zeigte sich, dass Umwelteinflüsse die Dämmwirkung der Brandschutzbeschichtung erheblich herabsetzen können. Weiterhin zeigte sich, dass ein Indiz für eine Leistungsfähigkeit eine resultierende aufgeschäumte Dicke ist. Eine zeitliche Entwicklung, insbesondere bei einem Vergleich von Linie 168 mit Linie 172 kann einen weiteren wichtigen Parameter darstellen. Einflüsse der Alterung auf die Bauteilbeanspruchung können durch das Prüfverfahren abgebildet werden.

### Liste der Bezugszeichen

- 110: Vorrichtung
- 112: Gehäuse
- 114: Innenraum
- 116: Wand
- 118: thermisches Isolationsmaterial
- 120: Seitenwand
- 122: feuerfestes Material
- 124: Sichtfenster
- 126: Handgerät
- 128: Tragevorrichtung
- 130: Griff
- 132: Befestigungselement
- 134: Wärmequelle
- 136: Prüföffnung
- 138: Infrarot-Wärmequelle
- 140: Sensor
- 142: Wärmebildkamera
- 144: Durchgangsöffnung
- 146: Schneidvorrichtung
- 148: Bauwerk
- 150: Dach
- 152: Stahlträger
- 154: Bauteil
- 156: Dämmschichtbildner
- 158: Prüfbereich
- 160: weiterer Bereich
- 162: erste Dämmschichtbildnerbeschichtung
- 164: zweite Dämmschichtbildnerbeschichtung
- 166: Oberfläche
- 168: Linie
- 170: Linie
- 172: Linie
- 174: Linie
- 176: Linie
- 178: Linie
- 180: Linie

## Patentansprüche

1. Verfahren zur Erfassung mindestens einer Eigenschaft eines Dämmschichtbildners (156), mit welchem ein Bauteil (154) eines Bauwerks (148) beschichtet ist, wobei das Verfahren die folgenden Schritte umfasst
a) Bereitstellen einer Vorrichtung (110) zur Erfassung mindestens einer Eigenschaft eines Dämmschichtbildners (156), mit welchem ein Bauteil (154) eines Bauwerks (148) beschichtet ist, wobei die Vorrichtung (110) umfasst:
- mindestens ein Gehäuse (112), wobei das Gehäuse (112) mindestens eine Prüföffnung (136) aufweist, welche eingerichtet ist, einen mit dem Dämmschichtbildner (156) beschichteten Prüfbereich (158) auf dem Bauteil (154) des Bauwerks (148) von weiteren Bereichen (160) des Bauteils (154) abzugrenzen;
- mindestens eine Wärmequelle (134), wobei die Wärmequelle (134) innerhalb des Gehäuses (112) angeordnet ist und wobei die Wärmequelle (134) eingerichtet ist, dem Prüfbereich (158) thermische Energie zuzuführen; und
- mindestens einen Sensor (140), wobei der Sensor (140) eingerichtet ist, eine durch die thermische Energie induzierte Änderung einer Eigenschaft des Dämmschichtbildners (156) zu erfassen;
b) Befestigen der Vorrichtung (110) an das Bauteil (154) des Bauwerks (148), derart, dass die Prüföffnung (136) des Gehäuses (112) einen mit dem Dämmschichtbildner (156) beschichteten Prüfbereich (158) auf dem Bauteil (154) des Bauwerks (148) von weiteren Bereichen (160) des Bauteils (154) abgrenzt;
c) Zuführen von thermischer Energie an dem Prüfbereich (158) mittels der Wärmequelle (134); und
d) Erfassen mindestens einer durch die thermische Energie induzierte Änderung einer Eigenschaft des Dämmschichtbildners (156) mittels des Sensors (140).

2. Verfahren nach dem vorhergehenden Anspruch, wobei in Schritt b) die Vorrichtung (110) an das Bauteil (154) des Bauwerks (148) mittels des Befestigungselements (132) befestigt wird.

3. Verfahren nach einem der beiden vorhergehenden Ansprüche, wobei nach Durchführen des Schritts c) oder d) der Prüfbereich (158) mit einem weiteren Dämmschichtbildner beschichtet wird.

4. Verfahren nach einem der vorhergehenden Ansprüche, wobei die gemäß Schritt a) bereitgestellte Vorrichtung (110) ein Handgerät (126) ist.

5. Verfahren nach einem der vorhergehenden Ansprüche, wobei mindestens eine Abmessung des Gehäuses (112) ausgewählt wird aus der Gruppe bestehend aus: einer Länge I des Gehäuses (112), einer Breite b des Gehäuses (112), einer Tiefe t des Gehäuses (112) 50 mm bis 500 mm, vorzugsweise von 80 mm bis 300 mm und besonders bevorzugt von 100 mm bis 200 mm.

6. Verfahren nach einem der vorhergehenden Ansprüche, wobei für die Prüföffnung (136) ein Durchmesser d von 10 mm bis 100 mm, vorzugsweise von 20 mm bis 80 mm und besonders bevorzugt von 30 mm bis 60 mm, ausgewählt wird.

7. Verfahren nach einem der vorhergehenden Ansprüche, wobei die Prüföffnung (136) des Gehäuses (112) mindestens eine zumindest teilweise umlaufende Schneidvorrichtung (146) umfasst, welche eine erste Dämmschichtbildnerbeschichtung (162) innerhalb des Prüfbereichs (158) von einer zweiten Dämmschichtbildnerbeschichtung (164) außerhalb des Prüfbereichs (158) thermisch und/oder mechanisch entkoppelt.

8. Verfahren nach einem der vorhergehenden Ansprüche, wobei der Sensor (140) ausgewählt wird aus der Gruppe bestehend aus: einem Thermoelement; einer Wärmebildkamera (142); einer Vorrichtung zur Schichtdickenbestimmung, insbesondere einer optischen Vorrichtung zur Schichtdeckenbestimmung, insbesondere einem Laser- und/oder Infrarotentfernungsmesser, insbesondere einer Vorrichtung zur Schichtdickenbestimmung nach einem Keilschnittverfahren.

9. Verfahren nach einem der vorhergehenden Ansprüche, wobei die Vorrichtung (110) weiterhin mindestens eine Absaugvorrichtung umfasst, welche Gase aus einen Innenraum (114) des Gehäuses (112) in eine äußere Umgebung des Gehäuses (112) transportiert.

10. Verfahren nach einem der vorhergehenden Ansprüche, wobei die Vorrichtung (110) weiterhin mindestens eine Gaszuführungsvorrichtung umfasst, welche Gase in den Innenraum (114) des Gehäuses (112) transportiert.

## Claims

1. Method for detecting at least one property of an intumescent paint (156), with which a structural part (154) of a building structure (148) is coated, wherein the method comprises the following steps
a) providing a device (110) for detecting at least one property of an intumescent paint (156), with which a structural part (154) of a building structure (148) is coated, wherein the device (110) comprises:
- at least one housing (112), wherein the housing (112) has at least one test opening (136), which is designed to separate a test area (158), coated with the intumescent paint (156), on the structural part (154) of the building structure (148) from other regions (160) of the structural part (154);
- at least one heat source (134), wherein the heat source (134) is arranged inside the housing (112) and wherein the heat source (134) is designed to supply thermal energy to the test area (158); and
- at least one sensor (140), wherein the sensor (140) is designed to detect a change in a property of the intumescent paint (156) that is induced by the thermal energy;
b) fastening the device (110) onto the structural part (154) of the building structure (148) in such a way that the test opening (136) of the housing (112) separates a test area (158), coated with the intumescent paint (156), on the structural part (154) of the building structure (148) from other regions (160) of the structural part (154);
c) supplying thermal energy at the test area (158) by means of the heat source (134); and
d) detecting at least one change in a property of the intumescent paint (156), induced by the thermal energy, by means of the sensor (140).

2. Method according to the preceding claim, wherein, in step b), the device (110) is fastened onto the structural part (154) of the building structure (148) by means of the fastening element (132).

3. Method according to one of the two preceding claims, wherein, after carrying out step c) or d), the test area (158) is coated with a further intumescent paint.

4. Method according to one of the preceding claims, wherein the device (110) provided according to step a) is a hand-held unit (126).

5. Method according to one of the preceding claims, wherein at least one dimension of the housing (112) is selected from the group consisting of: a length 1 of the housing (112), a width b of the housing (112), a depth t of the housing (112) 50 mm to 500 mm, preferably of 80 mm to 300 mm and particularly preferably of 100 mm to 200 mm.

6. Method according to one of the preceding claims, wherein a diameter d of 10 mm to 100 mm, preferably of 20 mm to 80 mm and particularly preferably of 30 mm to 60 mm, is selected for the test opening (136).

7. Method according to one of the preceding claims, wherein the test opening (136) of the housing (112) comprises at least one at least partially enclosing cutting device (146), which thermally and/or mechanically isolates a first intumescent paint coating (162) inside the test area (158) from a second intumescent paint coating (164) outside the test area (158) .

8. Method according to one of the preceding claims, wherein the sensor (140) is selected from the group consisting of: a thermocouple; a thermal imaging camera (142); a device for layer thickness determination, in particular an optical device for layer thickness determination, in particular a laser and/or infrared distance meter, in particular a device for layer thickness determination on the basis of a wedge cut method.

9. Method according to one of the preceding claims, wherein the device (110) also comprises at least one suction extraction device, which transports gases out of an interior space (114) of the housing (112) into an outer surrounding area of the housing (112).

10. Method according to one of the preceding claims, wherein the device (110) also comprises at least one gas supplying device, which transports gases into the interior space (114) of the housing (112).

## Revendications

1. Procédé de détection d'au moins une propriété d'un agent formateur de couche d'isolation (156) dont un composant (154) d'un bâtiment (148) est revêtu, le procédé comprenant les étapes suivantes
a) fournir un dispositif (110) destiné à détecter au moins une propriété d'un agent formateur de couche d'isolation (156) dont est revêtu un composant (154) d'un bâtiment (148), le dispositif (110) comprenant :
- au moins un boîtier (112), le boîtier (112) comportant au moins une ouverture de test (136) qui est conçue pour délimiter une zone de test (158), revêtue de l'agent de formation de couche isolante (156), sur le composant (154) du bâtiment (148) d'autres zones (160) du composant (154) ;
- au moins une source de chaleur (134), la source de chaleur (134) étant disposée à l'intérieur du boîtier (112) et la source de chaleur (134) étant conçue pour amener de l'énergie thermique à la zone de test (158) ; et
- au moins un capteur (140), le capteur (140) étant conçu pour détecter un changement de propriété, induit par l'énergie thermique, de l'agent de formation de couche d'isolation (156) ;
b) fixer le dispositif (110) au composant (154) du bâtiment (148) de telle sorte que l'ouverture de test (136) du boîtier (112) délimite une zone de test (158), revêtue de l'agent de formation de couche d'isolation (156), sur le composant (154) du bâtiment (148) d'autres zones (160) du composant (154) ;
c) amener de l'énergie thermique à la zone de test (158) au moyen de la source de chaleur (134) ; et
d) détecter au moins un changement de propriété, induit par l'énergie thermique, de l'agent de formation de couche d'isolation (156) au moyen du capteur (140).

2. Procédé selon la revendication précédente, à l'étape b) le dispositif (110) étant fixé au composant (154) du bâtiment (148) au moyen de l'élément de fixation (132).

3. Procédé selon l'une des deux revendications précédentes, après que l'étape c) ou d) a été réalisée, la zone de test (158) étant revêtue d'un autre agent de formation de couche d'isolation.

4. Procédé selon l'une des revendications précédentes, le dispositif (110) prévu selon l'étape a) étant un dispositif portatif (126).

5. Procédé selon l'une des revendications précédentes, au moins une dimension du boîtier (112) étant choisie dans le groupe comprenant : une longueur l du boîtier (112), une largeur b du boîtier (112), une profondeur t du boîtier (112) 50 mm à 500 mm, de préférence de 80 mm à 300 mm et de manière particulièrement préférée de 100 mm à 200 mm.

6. Procédé selon l'une des revendications précédentes, un diamètre d de 10 mm à 100 mm, de préférence de 20 mm à 80 mm et de manière particulièrement préférée de 30 mm à 60 mm, étant choisi pour l'ouverture de test (136).

7. Procédé selon l'une des revendications précédentes, l'ouverture de test (136) du boîtier (112) comprenant au moins un dispositif de coupe (146) au moins partiellement circonférentiel qui effectue un découplage thermique et/ou un désaccouplement mécaniquement entre un premier revêtement d'agent de formation de couche d'isolation (162) à l'intérieur de la zone de test (158) et un deuxième revêtement d'agent de formation de couche d'isolation (164) à l'extérieur de la zone de test (158).

8. Procédé selon l'une des revendications précédentes, le capteur (140) étant choisi dans le groupe comprenant : un thermocouple ; une caméra d'imagerie thermique (142) ; un dispositif de détermination d'épaisseur de couche, en particulier un dispositif optique de détermination d'épaisseur de couche, en particulier un télémètre laser et/ou infrarouge, en particulier un dispositif de détermination d'épaisseur de couche par un procédé de sectionnement à la lame.

9. Procédé selon l'une des revendications précédentes, le dispositif (110) comprenant en outre au moins un dispositif d'aspiration qui transporte des gaz d'un espace intérieur (114) du boîtier (112) jusque dans un environnement extérieur du boîtier (112).

10. Procédé selon l'une des revendications précédentes, le dispositif (110) comprenant en outre au moins un dispositif d'alimentation en gaz qui transporte des gaz jusque dans l'espace intérieur (114) du boîtier (112).
